(19) Europäisches Patentamt
European Patent Office
Office européen des brevets

(11) **EP 3 051 449 A1**

(12) **EUROPÄISCHE PATENTANMELDUNG**

(43) Veröffentlichungstag:
03.08.2016 Patentblatt 2016/31

(51) Int Cl.:
*G06F 19/12* (2011.01)     *G06F 19/26* (2011.01)

(21) Anmeldenummer: 15153052.4

(22) Anmeldetag: **29.01.2015**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Bayer Technology Services GmbH
51368 Leverkusen (DE)**

(72) Erfinder:
• **Neymann, Dr. Tobias
51465 Bergisch Gladbach (DE)**
• **Hebing, Lukas
44139 Dortmund (DE)**

• **Engell, Sebastian
58300 Wetter (DE)**

(74) Vertreter: **BIP Patents
c/o Bayer Intellectual Property GmbH
Alfred-Nobel-Straße 10
40789 Monheim am Rhein (DE)**

Bemerkungen:
Ein Antrag gemäss Regel 139 EPÜ auf Berichtigung
liegt vor. Über diesen Antrag wird im Laufe des
Verfahrens vor der Prüfungsabteilung eine
Entscheidung getroffen (Richtlinien für die Prüfung
im EPA, A-V, 3.).

(54) **Computerimplementiertes Verfahren zur Erstellung eines Fermentationsmodels**

(57) Die Anmeldung betrifft ein computerimplementiertes Verfahren zur Erstellung eines Models einer Bioreaktion
- Fermentations- oder Ganzzellkatalyseprozess - mit einem Organismus auf Basis von Messdaten.

EP 3 051 449 A1

**Beschreibung**

Die Erfindung betrifft ein Computer-implementiertes Verfahren zur Erstellung eines Models einer Bioreaktion - insb. Fermentation oder Ganzzellkatalyse - unter Verwendung eines Organismus. Organismus im Sinne der Anmeldung sind Kulturen aus pflanzlichen oder tierischen Zellen wie Säugetierzellen, Hefen, Bakterien, Algen, usw., die in Bioreaktionen eingesetzt werden.

Die sensorische Überwachung eines Fermentationsprozesses und Analyse von Proben aus einem Prozess z. B. mit Hilfe der Quality-by-Design Analytik-Automatisierungsplattform BaychroMAT® der Bayer Technology Services GmbH liefert verschiedene Informationen über den Zustand des Prozesses in dem Bioreaktor in Echtzeit. Typischerweise werden Zellenanzahl, Zellvitalität, Konzentrationen von Substraten, wie Kohlenstoffquellen (z. B. Glukose) oder $CO_2$, $O_2$, Aminosäuren, Produkte und Nebenprodukte (z. B. Laktat) oder Produktmerkmale ermittelt. Diese Daten können durch berechnete Daten und/oder Extrapolationen z. B. aus dem Stand der Technik ergänzt werden. Zusammen bilden diese Daten die Messdaten oder das Prozesswissen im Sinne der Anmeldung.

Hintergrundwissen zu dem Organismus bedeutet im Sinne der Anmeldung Wissen über die biochemischen Reaktionen des Organismus - spezifische und unspezifische Reaktionen - und insbesondere die zellinternen Reaktionen, bzw. Makroreaktionen zur Beschreibung der Organismus spezifischen Stoffwechselnetzwerke (auch SN oder metabolische Netzwerke genannt), die aus Substraten, Metaboliten (auch Knoten des metabolischen Netzwerks genannt), Produkten sowie den biochemischen Reaktionen zwischen ihnen bestehen. Diese biochemischen Reaktionen sind durch ihre:

(a) Stöchiometrie
(b) Reversibilität (unter biologischen Bedingungen),
(c) Einbindung in ein stöchiometrisches Netzwerk,
definiert.

Bisher werden die Messdaten hauptsächlich für die qualitative Überwachung des Prozesses eingesetzt.

Es bestand daher den Bedarf, ein Verfahren bereit zu stellen, das die volle Nutzung der ermittelten Daten für die Bereitstellung eines akkuraten Fermentationsmodells ermöglicht, das das Hintergrundwissen berücksichtigt und in einem modellbasierten, prädiktiven geschlossenen Regelkreis eingesetzt werden kann, mit dem Ziel Produktmenge, Produktmerkmale und Bildung von Nebenprodukten zu optimieren.

Typische Produktmerkmale im Sinne der Anmeldung sind beispielsweise Glykosylierungsmuster von Proteinen oder die Proteinintegrität, ohne sich darauf zu begrenzen.

Die modellbasierte Prozessführung von Fermentationen wird von Frahm et. al am Beispiel einer Hybridoma Zellkultur gezeigt (Frahm B, Lane P, Atzert H, Munack A, Hoffmann M, Hass VC, Portner R. 2002. Adaptive, Model-Based Control by the Open-Loop-Feedback-Optimal (OLFO) Controller for the Effective Fed-Batch Cultivation of Hybridoma Cells. Biotechnol. Prog. 18(5):1095-1103). Grundlegende Prozessgrößen werden hier modellbasiert gesteuert. Eine Integration von Produktmerkmalen findet hier nicht statt. Das mathematische Modell der Zelle wurde für diesen spezifischen Prozess entworfen und lässt sich nur mit großem Aufwand auf andere Organismen oder Stämme des gleichen Organismus übertragen. Hintergrundwissen in Form von zellinternen Reaktionen werden im Modell nicht explizit berücksichtigt. Eine Integration weiterer Messgrößen in das Modell kann hier nur mit sehr großem Aufwand erfolgen. Der Ansatz stellt somit eine individuelle Lösung dar, die weder auf andere Prozesse übertragbar ist, noch die volle Nutzung der ermittelten Daten ermöglicht.

Eine weitergehende Modellierung, die auch Produktmerkmale wie die Glykosylierung mit einschließt, findet sich in den Veröffentlichungen von Kontoravdi et. al. Das Modell, das den Hauptstoffwechsel beschreibt, bezieht kein Hintergrundwissen in Form von zellinternen Reaktionen mit ein und lässt sich auch nicht auf andere Organismen oder Stämme des gleichen Organismus übertragen. Eine Integration weiterer Messgrößen in das Modell kann hier nicht erfolgen [Kontoravdi C, Asprey SP, Pistikopoulos EN, Mantalaris A. 2007. Development of a dynamic model of monoclonal antibody production and glycosylation for product quality monitoring. Computers & Chemical Engineering 31(5-6):392-400.].

Die Modellierungen der Glykosylierung unter Einbindung des Nucleotidzuckerstoffwechsels von Jedrzejewski et al. und Jimenez et al. beziehen Hintergrundwissen in Form von Bilanzgleichungen interner Stoffwechselintermediate mit ein [Jedrzejewski PM, del Val, Ioscani Jimenez, Constantinou A, Dell A, Haslam SM, Polizzi KM, Kontoravdi C. 2014. Towards Controlling the Glycoform: A Model Framework Linking Extracellular Metabolites to Antibody Glycosylation. International journal of molecular sciences 15(3):4492-4522.; Jimenez del Val, Ioscani, Nagy JM, Kontoravdi C. 2011. A dynamic mathematical model for monoclonal antibody N-linked glycosylation and nucleotide sugar donor transport within a maturing Golgi apparatus. Biotechnology progress 27(6):1730-1743]. Bei einer Verwendung dieses Modells für die Prozessführung sind die Komplexität des gesamten Modells und die mangelnde Beobachtbarkeit zellinterner Stoffwechselintermediate aber nachteilig. Zudem lässt das Modell des Hauptstoffwechsels keine Übertragung auf andere Prozesse oder die Verwendung einer breiten Datenbasis zu.

Eine flexible Modellgenerierung für Bioprozesse wird von Leifheit et al. adressiert [Leifheit J, Heine T, Kawohl M, King R. 2007. Rechnergestützte halbautomatische Modellierung biotechnologischer Prozesse (Semiautomatic Modeling of Biotechnical Processes). at - Automatisierungstechnik 55(5)]. Die Modellgenerierung erfolgt unter Zuhilfenahme von Prozesswissen, allerdings ohne Hintergrundwissen. Die Prozedur ist für verschiedene Organismen oder Stämme des gleichen Organismus verwendbar. Basis sind hier Makroreaktionen, die der Anwender selbst vorgibt. Deren genaue Stöchiometrien werden in dem Verfahren ermittelt. Eine Integration weiterer Messgrößen ist somit mit einem signifikanten Anstieg der Komplexität des Verfahrens verbunden. Bei einer Verwendung von umfassenden Datengrundlagen, wie sie beispielsweise durch die BaychroMAT®-Plattform bereitgestellt wird, ist das Verfahren nicht mehr durchführbar. Eine Integration von Produktmerkmalen findet hier nicht statt.

Die Verwendung des Hintergrundwissens in Form von Makroreaktionen, die als Elementary Modes (EM) aus den bekannten metabolischen (stöchiometrischen) Netzwerken eines Organismus gewonnen werden, wird von Provost beschrieben [Provost A. 2006. Metabolic design of dynamic bioreaction models. Faculté des Sciences Appliquees, Universite catholique de Louvain, Louvain-la-Neuve, Louvain-la-Neuve, S. 81 ff., S.107 ff. S. 118 ff,]. Diese Methode ist für verschiedene Organismen oder Stämme des gleichen Organismus verwendbar. Die Auswahl der Makroreaktionen für das Prozessmodell erfolgt unter Verwendung von Prozesswissen. Jedoch werden Prozessabschnitte definiert für die eine vordefinierte Anzahl von Makroreaktionen separat zufällig ausgewählt werden. Das beschriebene Verfahren liefert hier eine von vielen möglichen Kombinationen von Elementary Modes. Die Anzahl der Makroreaktionen, und somit die Modellkomplexität, ist festgelegt und nicht veränderbar. Das Verfahren ergibt separate Modelle für jeden Prozessabschnitt. Die Auswahl der Kinetiken der einzelnen Makroreaktionen erfolgt unter Berücksichtigung der Stöchiometrie der gewählten Makroreaktionen. Die Parameter der Kinetiken (Modellparameter) werden jedoch nicht an die Prozessdaten angepasst. Stattdessen bildet die Verwendung der separaten Prozessabschnittsmodelle die Änderungen in den Prozessdaten ab. Die zufällige Auswahl der Reaktionen kann zwar auch auf Basis einer umfassenden Datengrundlage erfolgen, allerdings können durch den beschriebenen Ansatz zur Auswahl der Kinetiken und die ausgewählten Kinetiken den Verlauf des Prozesses bzw. das Verhalten des Organismus im Prozess nicht abbilden. Die Verwendung mehrerer Prozessabschnittsmodelle führt zudem zu einer unnötigen Komplexitätssteigerung für die Prozessführung. Auch findet hier eine Integration von Produktmerkmalen nicht statt.

Es bestand daher der Bedarf ein Verfahren bereit zu stellen, das die schnelle und effiziente Bereitstellung eines Modells auf Basis von Prozesswissen und -Messdaten und die Optimierung des Produktumsatzes und der kritischen Produktmerkmale unter Berücksichtigung von Hintergrundwissen ermöglicht und die oben genannten Nachteile nicht aufweist.

Die Aufgabe wurde durch ein Verfahren zur Erstellung eines Models einer Bioreaktion mit einem Organismus in einem Bioreaktor wie folgend beschrieben gelöst.

Gegenstand der Anmeldung ist ein computerimplementiertes Verfahren zur Erstellung eines Models einer Bioreaktion - insb. Fermentation oder Ganzzellkatalyse - mit einem Organismus, das folgende Schritte umfasst:

a. Ausgewählte Stoffwechselwege des Organismus, deren Stöchiometrie- sowie Reversibilitätseigenschaften werden als Hintergrundwissen in das Verfahren eingegeben. Mit anderen Worten werden ein oder mehrere metabolische Netzwerke des Organismus in das Verfahren eingegeben. Elementary Modes (EMs) werden aus dieser Eingabe berechnet.

b. Die EMs werden in einer Matrix $K$ zusammengefasst, wobei die EMs die Stoffwechselwege aus a) in Makroreaktionen zusammenfassen. Diese Matrix $K$ enthält hiermit die Stöchiometrie und die Reversibilitätseigenschaften aller möglichen Makroreaktionen aus dem Hintergrundwissen.

c. Die Messdaten (auch Prozesswissen genannt) zur Bioreaktion mit dem Organismus werden eingegeben.

d. Mit Hilfe einer Interpolationsmethode werden auf Basis der eingegebenen Messdaten aus c) die für den Organismus spezifischen Raten - Ausscheide- und Aufnahmeraten von einer oder mehreren Eingangsgrößen und Ausgangsgrößen - der eingegebenen Stoffwechselwege berechnet. Bevorzugt werden auch Wachstumsraten, besonders bevorzugt auch Absterberaten des Organismus berechnet.

e. Relevante Makroreaktionen werden in Form eines Subsets der Elementary Modes aus a) ausgewählt durch

i. datenunabhängige Vorreduktion der Anzahl der EMs aus a),

ii. Auswahl des Subsets basiert auf den Messdaten aus c) und / oder einer oder mehrerer Raten aus d), bevorzugt basierend auf einer oder mehreren der Raten aus d), aus der vorreduzierten Anzahl der EMs aus i.,

iii. Das Subset wird in einer Matrix L zusammengefasst, die die Stöchiometrie und Reversibilität der ausgewählten Makroreaktionen des Subsets aus ii. beschreibt.

f. Mit Hilfe einer Interpolationsmethode werden auf Basis der eigegebenen Messdaten aus c) und / oder der Raten aus d), die Reaktionsraten der Makroreaktionen des Subsets berechnet.

g. Die Kinetiken der Makroreaktionen des Subsets werden auf Basis der Stöchiometrie der Makroreaktionen des Subsets aus e) iii. und einer individuellen Anpassung basierend auf einer Analyse der Reaktionsraten aus f) entworfen; dadurch werden die Modellparameter definiert.

h. Optional erfolgt aus den Kinetiken aus g) eine erste Anpassung der Modellparameterwerte für jede Makroreaktion separat an die berechneten Reaktionsraten aus f).

i. Optional werden die Schritte g) und h) wiederholt bis eine vordefinierte Anpassungsgüte erreicht wird.

j. Die Modellparameterwerte werden an die Messdaten aus c) angepasst.

k. Die Matrix L, die Kinetiken aus g) und die Modellparameterwerte aus j) bilden das Modell und werden ausgegeben.

Die erfindungsgemäße Modellierung der Bioreaktion baut im Wesentlichen auf der Annahme repräsentativer Makroreaktionen auf, die interne Stoffwechselvorgänge vereinfacht darstellen. Für die Auswahl der Reaktionen wird sowohl biochemisches Hintergrundwissen, als auch Prozesswissen benötigt.

Im ersten Schritt des Verfahrens erfolgt die Eingabe der Reaktionen des metabolischen Netzwerkes, deren Stöchiometrie und Reversibilitätseigenschaft durch den Nutzer über eine Nutzeroberfläche oder idealerweise automatisch durch die Selektion eines Organismus und seiner hinterlegten Stoffwechselwege aus einem Datenbankmodul, in dem das Hintergrundwissen zum Organismus gespeichert ist. Das metabolische Netzwerk (im Stand der Technik auch stöchiometrische Netzwerk benannt) und die Eigenschaften seiner einzelnen Reaktionen stellen das Hintergrundwissen des Organismus dar. Bevorzugt enthält das metabolische Netzwerk Reaktionen, die das Zellwachstum beschreiben, z. B. in Form einer vereinfachten Reaktion von internen Metaboliten zur externen Biomasse.

Dann werden aus den eingegebenen, in einem oder mehreren stöchiometrischen Netzwerken zusammengefassten, Stoffwechselwegen des Organismus Elementary Modes berechnet. Jeder Elementary Mode ist eine Linearkombination von Reaktionsraten aus den Stoffwechselwegen - d. h. internen und externen Reaktionen des metabolischen Netzwerkes, bei der sowohl die "steady state" Bedingung für interne Metabolite erfüllt als auch die Reversibilität bzw. Irreversibilität von Reaktionen berücksichtigt sind. Bei Linearkombinationen von Reaktionen, die die "steady state" Bedingung für interne Metabolite berücksichtigen, können sich keine internen Metabolite akkumulieren.

Eine interne Reaktion im Sinne der Anmeldung erfolgt ausschließlich innerhalb der Zelle.

Eine externe Reaktion im Sinne der Anmeldung enthält mindestens eine Komponente außerhalb der Zelle, typischerweise mindestens eine Eingangsgröße und/oder mindestens eine Ausgangsgröße (Produkt Nebenprodukt, usw.). Durch eine Externalisierung einer internen Komponente, d. h. durch eine Klassifizierung einer eigentlich internen Komponente als Ein- oder Ausgangsgröße, ist es möglich, die mit der externalisierten, internen Komponenten verbundene interne Reaktion als externe Reaktion zu modellieren und somit die "steady-state" Bedingung für interne Metabolite in diesem Fall zu umgehen.

Eine Makroreaktion im Sinne der Anmeldung fasst alle Reaktionen zusammen, die von einer oder mehreren Eingangsgrößen zu einer oder mehreren Ausgangsgröße führen. Jeder Elementary Mode beschreibt somit eine Makroreaktion. Im Vergleich zu der Methode von Leifheit. et al werden im Sinne der Anmeldung die Makroreaktionen auf Basis des eingegebenen Hintergrundwissen ermittelt.

Die Elementary Modes (EMs) werden in einer Matrix E zusammengefasst, vorzugsweise in einem Modul zum Matrixaufbau, das mit einem entsprechenden Algorithmus konfiguriert ist. Bekannte Algorithmen können zum Aufbau der Elementary Modes Matrix verwendet werden. METATOOL wird als Beispiel genannt, ohne sich darauf zu begrenzen: [Pfeiffer T, Montero F, Schuster S. 1999. METATOOL: for studying metabolic networks. Bioinformatics 15(3):251-257.]

Mit METATOOL wird eine erste Matrix E, die die eingegebenen internen und externen Reaktionen beschreibt, erzeugt.

In Schritt b) wird mit Hilfe der (externen) stöchiometrischen Matrix $N_p$ aus der Matrix (E) eine Matrix bestehend aus möglichen Makroreaktionen $K$ erzeugt.

$$K = N_p \cdot E \quad (\text{Formel 1})$$

Die Transformation der Matrix E nach $K$ ist aus Provost bekannt [Provost A. 2006. Metabolic design of dynamic bioreaction models. Faculté des Sciences Appliquees, Universite catholique de Louvain, Louvain-la-Neuve, Louvain-la-Neuve, S. 81].

Die Spaltenvektoren der Matrix $K$ beschreiben die Makroreaktionen. Die Zeilenvektoren beschreiben die Komponenten der Makroreaktionen (Eingangs- und Ausgangsgrößen). In die Matrix $K$ wird die Stöchiometrie der Makroreaktion eingetragen.

Jede im Sinne des metabolischen Netzwerks mögliche Reaktionsrate kann als positive Linearkombination dieser Makroreaktionen dargestellt werden.

Die Verwendung von EMs als Basis eines Prozessmodells ist im Stand der Technik bekannt (z. B. aus Provost A. 2006. Metabolic design of dynamic bioreaction models. Faculté des Sciences Appliquees, Universite catholique de Louvain, Louvain-la-Neuve, Louvain-la-Neuve S. 87, S. 118 ff. und Gao, J. et al. (2007). Dynamic metabolic modeling for a MAB bioprocess. Biotechnology progress, 23(1), 168-181).

In einem weiteren Schritt c) werden die verfügbaren Messdaten (Prozesswissen) zur Bioreaktion mit dem Organismus eingegeben.

Diese Eingabe kann manuell durch den Nutzer oder automatisch erfolgen, wie z. B. durch Selektion aus einem Datenbankmodul zur Speicherung von Messdaten und Transfer der selektierten Daten in ein Datenanalysemodul, das mit dem Datenbankmodul verbunden ist.

Aus diesen Messdaten werden im Schritt d) die zellspezifischen Ausscheide- und Aufnahmeraten von Substraten und (Neben)produkten - zusammen spezifische Raten $\underline{q}$ (t) - genannt, sowie optional die Wachstums- und Absterberaten des Organismus ($\mu(t)$, $\mu d(t)$), mit Hilfe einer Interpolationsmethode berechnet. Die berechneten Raten $\underline{q}(t)$, $\mu(t)$, $\mu_d(t)$ geben Auskunft über das zu beobachtende dynamische Verhalten des Organismus über der Zeit.

Für die Berechnung der o. g. Raten kann eine oder mehrere unterschiedliche Interpolationsmethode(n) in Kombination verwendet werden.

Exemplarisch beschreibt Leifheit et al. die Ermittlung der zeitlichen Änderungen von Messgrößen - z. B. die der Gesamtzellzahl $\frac{dX_t}{dt}(t)$, die der vitalen Zellzahl $\frac{dX_v}{dt}(t)$, oder die von anderen Medienkonzentrationen $\frac{dC_i}{dt}(t)$ - aus Messdaten mit Hilfe von Spline-interpolierten Messdaten [Leifheit, J., Heine, T., Kawohl, M., & King, R. (2007). Rechnergestützte halbautomatische Modellierung biotechnologischer Prozesse (Semiautomatic Modeling of Biotechnical Processes). at-Automatisierungstechnik, 55(5), 211-218]. Diese Methode wird hiermit per Referenz in der Anmeldung integriert.

Die o. g. Raten $\underline{q}(t)$, $\mu(t)$, $\mu_d(t)$ werden aus diesen zeitlichen Änderungen mit Hilfe einer weiteren Interpolationsmethode berechnet:

Beispielsweise können aus $\frac{dX_t}{dt}(t)$ die Wachstumsrate des Organismus $\mu(t)$ aus Spline-interpolierten Werten der Gesamtzellzahl $X_t(t)$ und der Lebendzellzahl $X_v(t)$ mit Formel 2 berechnet werden:

$$\frac{dX_t}{dt}(t) = -D(t) \cdot X_t(t) + \mu(t) \cdot X_v(t) \qquad \text{(Formel 2)}$$

Wobei $D(t)$ die Verdünnungsrate ist.

Die Absterberate $\mu_d(t)$ kann bei bekanntem Verlauf von $\mu(t)$ aus dem Verlauf von $X_v(t)$ und dem Verlauf der zeitlichen Änderung der vitalen Zellzahl $\frac{dX_v}{dt}(t)$ mit Formel 3 berechnet werden:

$$\frac{dX_v}{dt}(t) = -D(t) \cdot X_v(t) + (\mu(t) - \mu_d(t)) \cdot X_v(t) \qquad \text{(Formel 3)}$$

Die Berechnung der spezifischen Raten einer anderen Komponente i $q_i(t)$ kann aus Splineinterpolierten Werten der Lebendzellzahl $X_v(t)$ und der Konzentration der Komponente $c_i(t)$, sowie dem Verlauf der zeitlichen Änderung $\frac{dC_i}{dt}(t)$ mit Formel 4 erfolgen:

$$\frac{dC_i}{dt}(t) = D(t) \cdot \left( C_{i,in} - C_i(t) \right) + q_i(t) \cdot X_v(t) \qquad \text{(Formel 4)}$$

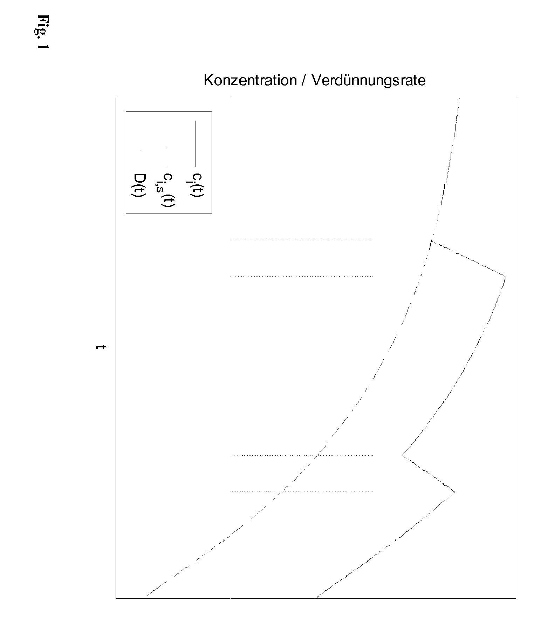

In einer bevorzugten Ausführungsform des Verfahrens werden die Messdaten aus Schritt c) vor der ersten Interpolation folgendermaßen aufbereitet: Um alle nicht von den Zellen verursachten Konzentrationsänderung zu berücksichtigen und aus den Messdaten einen stetigen Verlauf der Konzentrationsänderungen zu erhalten, werden die Messdaten verschoben (in der Anmeldung shiften genannt). Diese Aufbereitung (shiften) der Messdaten verhindert eine sprunghafte Änderung der berechneten spezifischen Raten beim An- oder Abstellen eines Feeds insbesondere in einem Fed-Batch Prozess. Figur 1 zeigt das Aufbereitung/Shiften der Messdaten im Sinne dieser Anmeldung.

In einer besonderen Ausführungsform des Verfahrens werden die aufbereiteten Daten dann zur Berechnung eines Gradienten der Gesamtzellzahl $\frac{dX_t}{dt}\big|_s(t)$ mit der Methode von Leifheit et al. verwendet. Dieser wird mit einer Spline-Interpolation gemäß Differentialgleichung 5 angenähert:

$$\frac{dX_t}{dt}\Big|_s(t) = \mu(t) \cdot X_v(t) \qquad \text{(Formel 5)}$$

Besonders bevorzugt wird die Lysis in der Berechnung anhand eines Lysis-Faktors $K_l$, einbezogen (Formel 6). Dieser kann z. B. als konstant über dem Prozessverlauf angenommen werden.

$$\frac{dX_t}{dt}\Big|_s = \mu(t) \cdot X_v(t) - K_l \cdot (X_t(t) - X_v(t)) \qquad \text{(Formel 6)}$$

Das Absinken der geshifteten Gesamtzellzahl $X_{t,s}(t)$ kann so durch Lysis erklärt werden, wodurch negative Werte für die Wachstumsraten $\mu(t)$ vermieden werden können.

Bevorzugt werden die aufbereiteten Daten auch für die Berechnung der Absterberate $\mu_d(t)$ verwendet.

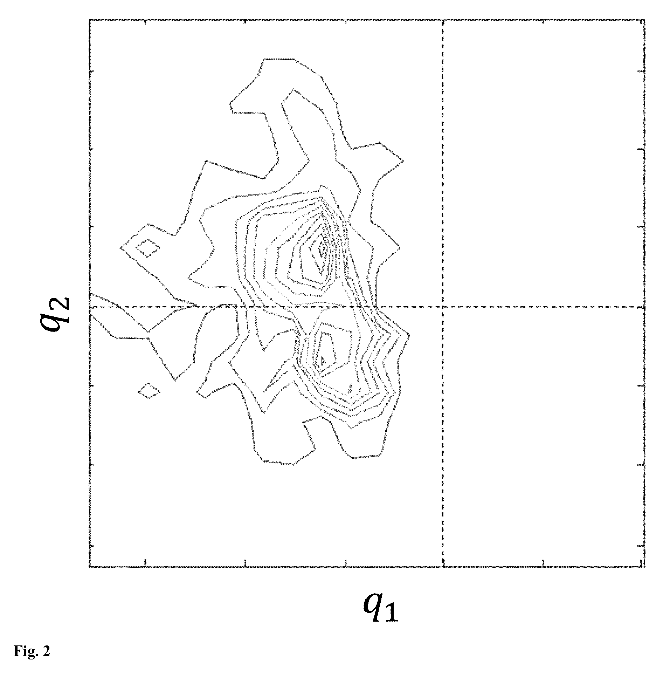

Bevorzugt werden die möglichen Kombinationen spezifischer Raten $q(t)$ in einem Flux-Map Diagramm wie beispielhaft in Figur 2 dargestellt. Diese Darstellung ermöglicht einem schnellen Plausibilitätstest der berechneten spezifischen Raten $q(t)$. Die Höhenlinien geben hier an, welche Bereiche physiologisch wichtig sind.

Wenn die spezifischen Raten $q(t)$, und optional die weiteren Raten $\mu(t)$, $\mu_d(t)$ unterschiedliche Größenordnungen und Einheiten besitzen, werden diese üblicherweise mit Hilfe von Vereinfachungen zu einem spezifischen Ratenvektor $\tilde{q}(t)$ mit gleichen Einheiten zusammengefasst. Beispielsweise wird die spezifische Rate eines Makromoleküls, das in Gramm [g] gemessen wird, die Einheit $\left[\frac{g}{Cell \cdot h}\right]$ besitzen. Wenn die Zusammensetzung dieses Makromoleküls geschätzt wird, z. B. basierend auf seinem C-mol-Gehalt, lässt sich seine spezifische Rate von [g] auf [C - mol] verändert darstellen, so dass die spezifische Rate die Einheit $\left[\frac{C-mol}{Cell \cdot h}\right]$ besitzt.

Die spezifischen Raten $\tilde{q}(t)$ bilden eine der Grundlagen für den weiteren Schritt e) des Verfahrens, nämlich die Auswahl der relevanten Makroreaktionen.

Im Schritt e) wird ein Subset (L) der EMs auf Basis der Daten ausgewählt, das möglichst viele Kombinationen der spezifischen Raten $\tilde{q}(t)$ aus d) und / oder Messdaten aus c) abbildet, wobei das dynamische Verhalten der Zellen über der Zeit mit einer möglichst geringen Anzahl von EMs ausreichend gut wiedergeben werden soll.

Die Selektion von EMs verkleinert den Lösungsraum im Vergleich zum original EMs-Set (K) aus a), aber enthält weiterhin den ermittelten physiologisch wichtigen Bereich der Zellen.

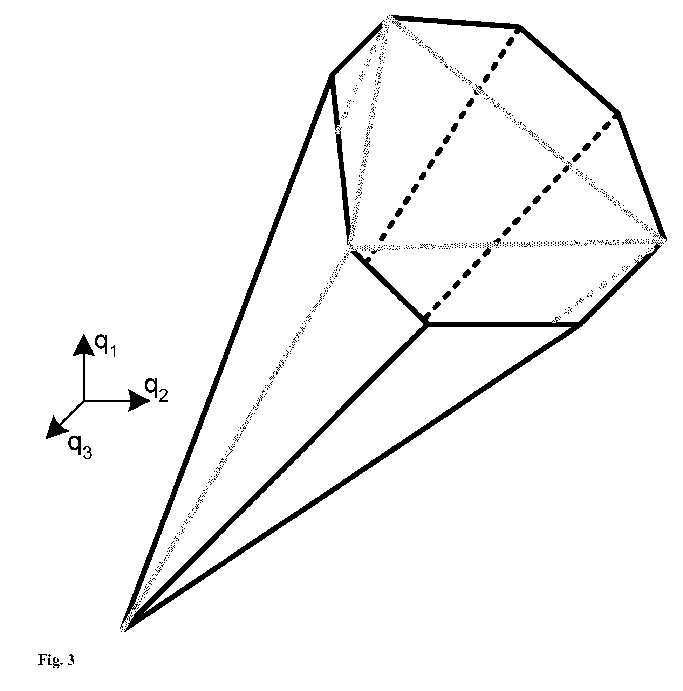

Figur 3 zeigt eine Darstellung des Lösungsraums, wobei das originale Set von EMs (K) zu einem Subset (L) reduziert wird.

Für diesen weiteren Schritt werden die berechneten spezifischen Raten $\tilde{q}(t)$ üblicherweise in ein Modul zur Auswahl der relevanten Makroreaktionen transferiert, das mit entsprechenden Algorithmen konfiguriert ist.

Methoden für eine solche Auswahl sind z. B. aus Provost et al. und Soons et al. bekannt [Provost A. 2006. Metabolic design of dynamic bioreaction models. Faculté des Sciences Appliquees, Universite catholique de Louvain, Louvain-la-Neuve, Louvain-la-Neuve ; Soons, Z. I. T. A., Ferreira, E. C., & Rocha, I. (2010). Selection of elementary modes for bioprocess control.].

Soons et al. beschreiben die Bildung eines EM-Subsets aus einer kleinen Anzahl von EMs durch zufällige Auswahl. Hierbei soll die Differenz zwischen den berechneten spezifischen Raten $\tilde{q}(t)$ und den mit diesem Subset darstellbaren Raten minimiert werden. Bei einer großen Zahl von EMs ist jedoch die zufällige Auswahl ineffektiv, da die Anzahl der möglichen Kombinationen sehr stark wächst. Beispielsweise gibt es bei der Auswahl von 10 Reaktionen aus einem Set von 20.000 EMs mehr als $2,8 \cdot 10^{36}$ Kombinationen. Die Wahrscheinlichkeit, dass die optimale Kombination gefunden wird ist hier sehr gering.

Provost beschreibt ein Verfahren, bei dem für verschiedene spezifische Werte von $\tilde{q}(t_i)$ $i = 1, \dots, n$ alle möglichen positiven Linearkombinationen von Elementary Modes ermittelt werden. Von diesen zahlreichen möglichen Kombinationen wird anschließend eine Kombination zufällig ausgewählt. Dieses Verfahren verwendet jeweils nur einen Vektor $\tilde{q}(t_i)$ und nicht den gesamten Verlauf über der Zeit. Eine Auswahl von EMs für den gesamten Prozess ist deshalb nicht möglich. Mit der zufälligen Auswahl lässt sich zwar der Vektor $\tilde{q}(t_i)$ darstellen, inwiefern der Rest des Prozesses hiermit dargestellt werden kann, wird jedoch nicht ermittelt. Ein weiterer Nachteil dieses Verfahrens ist, dass ein Vektor $\tilde{q}(t_i)$,

der nicht im Lösungsraum aller EMs liegt, nicht verwendet werden kann. Eine Näherungslösung lässt sich nicht ermitteln. Dies ist vor allem bei mit Unsicherheit behafteten Messungen und spezifischen Raten ein großer Nachteil.

Leighty, R. et al. beschreibt eine weitere Methode in der die Messwerte (Konzentrationsmessungen) direkt durch eine lineare Schätzung von volumetrischen Reaktionsraten über der Zeit approximiert werden. Durch Lösen eines linearen Optimierungsproblems kann der Verlauf der Reaktionen schnell geschätzt werden [Leighty, R. W., & Antoniewicz, M. R. (2011), Dynamic metabolic flux analysis (DMFA): a framework for determining fluxes at metabolic non-steady state. Metabolic engineering, 13(6), 745-755]. Diese Methode bezieht sich lediglich auf reversible Makroreaktionen (wie den in der Quelle genannten "Free Fluxes"), zudem können Verdünnungseffekte (also Konzentrationsänderungen, die nicht durch die Zellen verursacht werden) nicht berücksichtigt werden. Stimmen die Dimensionen der Makroreaktionen und der Messwerte nicht überein können diese Messwerte nicht zur Schätzung von Reaktionsraten verwendet werden. Dies ist zum Beispiel der Fall wenn das Zellwachstum in Form von Bildung externer Biomasse Teil der Makroreaktionen ist und aus den Messwerten nur die Zelltrockenmasse bekannt ist. In dieser Form ist diese Methode daher nicht auf die Anwendung von irreversiblen Makroreaktionen und Fed-Batch Prozesse geeignet.

Verwendet man das Konzept von Leighty et al., das hiermit per Referenz in diese Anmeldung integriert wird, mit den erfindungsgemäß aufbereiteten (geshifteten) Daten, lässt sich diese Methode jetzt auch auf Fed-Batch Prozesse anwenden. Darüber hinaus kann durch das Hinzufügen einer unteren Grenze für die Reaktionsraten der Makroreaktionen als Randbedingung des linearen Optimierungsproblems die Methode auch für irreversible Reaktionen - wie die Elementary Modes- verwendet werden. Stimmen die Dimensionen der Makroreaktionen und der Messwerte nicht überein kann über geeignete Korrelationen die Dimension der Messwerte an die der Makroreaktionen angepasst werden. Diese Kombination der linearen Schätzung nach Leighty et al. mit den Verbesserungen aus diesem Anspruch wird im Folgenden als "lineare Schätzung von Reaktionsraten ausgewählter Makroreaktionen" bezeichnet.

Es lässt sich so überprüfen, ob sich mit den gewählten Makroreaktionen eines Subsets $L$ des originalen EM-Sets $K$ die gemessenen Daten hinreichend darstellen lassen. Die hier errechnete finale Summe der kleinsten Fehlerquadrate des linearen Optimierungsproblems gibt an wie gut die Messdaten mit dem Subset dargestellt werden können. Je besser die Messdaten wiedergegeben werden können, desto besser ist das Subset $L$. Diese Verbesserung ist insbesondere für die Modellierung von Fed-Batch Prozessen vorteilhaft, da eine Überprüfung der Güte eines Subsets schnell auch ohne eine eventuell fehlerbehaftete vorherige Bestimmung der spezifischen Raten möglich ist.

Bevorzugt erfolgt die Auswahl der relevanten Makroreaktionen mit den folgenden Schritten aus Anspruch e):

In einem ersten Schritt i) erfolgt eine datenunabhängige Vorreduktion des Sets der EMs beschrieben durch die Matrix $K$ durch sukzessives Weglassen einzelner Reaktionen, die anderen Reaktion sehr ähnlich sind. Diese Vorreduktionen erfolgt durch eine geometrische Reduktion, z. B. mittels einer Kosinus-Ähnlichkeit. Diese Vorreduktion, die zu einer Volumenreduktion des Lösungsraums führt, wird wiederholt, bis eine vordefinierte Anzahl erreicht ist. Die gewünschte Anzahl wird üblicherweise für das Verfahren im Vorfeld definiert. Als Kontrollvariable kann das Volumen des Lösungsraumes verwendet werden. Überraschenderweise wurde festgestellt, dass eine Volumenreduktion von 90 bis 98 %, bevorzugt 92 bis 95% des aufgespanntes Volumen, im Vergleich zum Originalvolumen möglich ist, ohne wesentliche Beschränkung des Lösungsraums aber mit einer deutlichen Reduktion der Anzahl der Makroreaktionen.

Zur Auswahl des EM-Subsets L auf Basis der Messdaten aus dem vorreduziertem Set der EMs kann dann eine der oben beschriebenen Methoden, bevorzugt die "lineare Schätzung von Reaktionsraten ausgewählter Makroreaktionen" verwendet werden.

In einer weiteren und bevorzugten Ausführungsform des Verfahrens wird zur Auswahl der relevanten Makroreaktionen, d. h. zur Auswahl des EM-Subsets L ein evolutionärer, insbesondere ein genetischer, Algorithmus verwendet. Ein solcher Algorithmus ist z. B. aus Baker et al. bekannt [Syed Murtuza Baker, Kai Schallau, Björn H. Junker. 2010. Comparison of different algorithms for simultaneous estimation of multiple parameters in kinetic metabolic models. J. Integrative Bioinformatics:-1-1.]. Besonders bevorzugt kann ein genetischer Algorithmus verwendet werden, in dessen Zielfunktion für verschiedene Kombinationen von EMs die finale Summe der kleinsten Fehlerquadrate mit der Methode "lineare Schätzung von Reaktionsraten ausgewählter Makroreaktionen" berechnet wird. Alternativ kann auch eine zufällige Auswahl verwendet werden. Nach Abschluss des Schritt ii) beinhaltet die Matrix $L$ die notwendigen Makroreaktionen (Schritt iii).

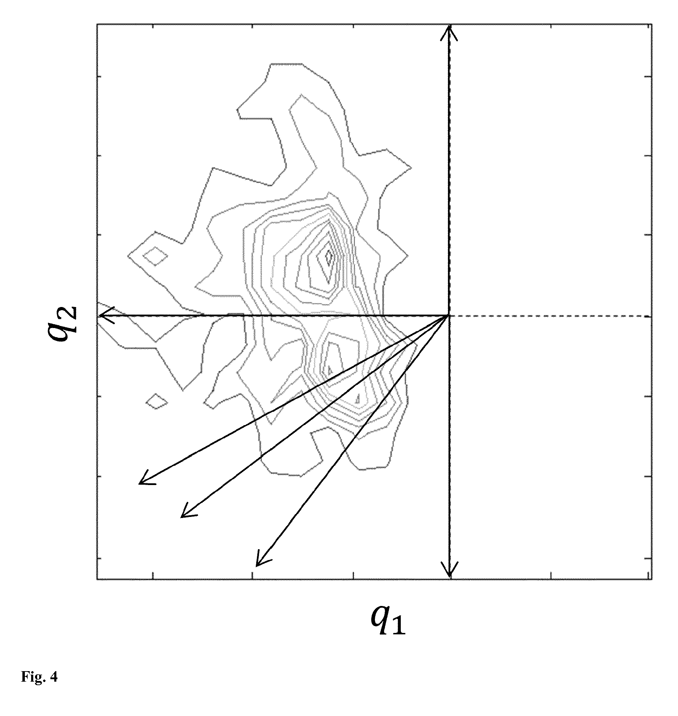

Zur Prüfung der Validität des EM-Subsets $L$ kann auch hier die Flux Map herangezogen werden. Figur 4 zeigt die Flux Map mit der Projektion eines Subsets von sechs EMs. Ist der EM-Subset $L$ valide, befinden sich die Messdaten weiterhin innerhalb des EM-Subsets $L$. Diese Darstellung ermöglicht eine schnelle Kontrolle der Validität der Auswahl.

In einem weiteren Schritt f) werden mit Hilfe einer Interpolationsmethode auf Basis der eingegebenen Messdaten aus c) die Reaktionsraten der Makroreaktionen des Subsets $L$ berechnet. Bevorzugt werden auf Basis der Methode "lineare Schätzung von Reaktionsraten ausgewählter Makroreaktionen" die Reaktionsraten der Makroreaktionen berechnet. Diese werden als $r(t)$ bezeichnet.

Im Schritt g) des Verfahrens werden die Kinetiken der Makroreaktionen entworfen. Die ermittelten Kinetiken sollen die dynamischen Einflüsse von Prozessbedingungen auf die jeweiligen Reaktionsraten quantifizieren. Aus den Kinetiken ergeben sich die zu bestimmenden Modellparameter.

Grundlage der Kinetiken bilden einerseits die Stöchiometrie der Makroreaktionen und andererseits Erkenntnisse aus Analysen, die mit den in f) berechneten Reaktionsraten $r(t)$ gewonnen werden.

Die Ermittlung von Kinetiken aus den Reaktionsstöchiometrien wird von Provost [Provost A. 2006. Metabolic design of dynamic bioreaction models. Faculté des Sciences Appliquees, Universite catholique de Louvain, Louvain-la-Neuve, S. 126] oder von Gao et al. [Gao, J., Gorenflo, V. M., Scharer, J. M., & Budman, H. M. (2007). Dynamic metabolic modeling for a MAB bioprocess. Biotechnology progress, 23(1), 168-181] beschrieben, deren Methode hiermit in der Anmeldung per Referenz integriert werden. In diesen Methoden werden der Substratlimitierungen des Monod-Typs für die jeweiligen Substrate einer Reaktion verwendet. Obwohl Provost oder Gao dies nicht beschreiben, sind Inhibierungen durch toxische Produkte ebenfalls mit dieser Methode aus der Reaktionsstöchiometrie ableitbar.

Bevorzugt werden weitere kinetische Terme (wie Inhibierungen, die nicht aus der Stöchiometrie ableitbar sind, und / oder Bioreaktionsbedingungen wie z. B. der pH-Wert, die Reaktortemperatur, Partialdrücke, usw.) aus einer Datenanalyse gewonnen. Hierfür können die bereits erwähnten erweiterten Prozessinformationen für den Entwurf der Kinetik verwendet werden. Als erweiterte Prozessinformationen sind die spezifische Raten $q(t)$ und die bevorzugten Wachstums- und Absterberaten $\mu(t)$, $\mu_d(t)$ aus d), sowie die Reaktionsraten einzelner Makroreaktionen r(t) aus f) oder auch Wertebereiche, in denen sich die Reaktionsraten $r(t)$ befinden können, gemeint. Diese Bereiche sind z. B. aus den berechneten Reaktionsraten und der Berücksichtigung von Unsicherheiten ableitbar. Bevorzugt werden alle erweiterten Prozessinformationen in dem erfindungsgemäßen Verfahren berücksichtigt.

In einem optionalen Schritt h) werden Modellparameterwerte der Kinetiken an die in f) ermittelten Reaktionsraten der Makroreaktionen r(t) angepasst. Dieses wird im Folgenden als Modellparameterwertschätzung bezeichnet. Auf ein numerisches Lösen einer oder mehrerer Differentialgleichungen nach der Formel 2 bis 4 in diesem Schritt kann verzichtet werden; die Modellparameterwerte können in unabhängigen Gruppen üblicherweise mit 3 bis 10 Parametern separat für jede Makroreaktion angepasst werden. Diese für jede Makroreaktion separate Modellparameterwertschätzung ist für die Schritte i) und j) besonders vorteilhaft, da sie einerseits schnell durchführbar ist und anderseits Startwerte für die Anpassung der Modellparameterwerte an Messdaten aus c) liefert.

In einem optionalen Schritt i) werden die in g) gewählten Kinetiken der Makroreaktionen auf ihre Anpassungsgüte hin überprüft. Grundlage ist die Anpassungsgüte der Modellparameterwertschätzung aus Schritt h). Die Überprüfung der Anpassungsgüte ist aufgrund ihrer schnellen Durchführbarkeit besonders vorteilhaft. Bei einer nicht zufriedenstellenden Anpassungsgüte können die Schritte g) und h) wiederholt werden, bis eine vordefinierte Anpassungsgüte erreicht wird.

In einem weiteren Schritt j) kann die Anpassung der Parameterwerte der Kinetiken aus g) an die Messdaten aus c) nach einer für Anpassungen üblichen Methode erfolgen. Bevorzugt werden für diese Anpassung die Startwerte aus Schritt h) verwendet. Die Modellparameterwertanpassung erfolgt unter Einbeziehung der o. g. Differentialgleichungen (Formel 2 bis 4), z. B. mit Hilfe eines *multiple-Shooting* Algorithmus.

Bevorzugt können Produktmerkmale in das Modell integriert werden. Besonders bevorzugt kann dies für Produktmerkmale, die von der Konzentration von Neben- oder Zwischenprodukten abhängen, eingeführt werden. Konzentrationen von Nebenprodukten, die externe Komponenten des in a) eingegebenen metabolischen Netzwerkes sind, sind bereits in das Modell integriert und können berechnet werden. Bei Bedarf lassen sich allerdings auch andere Neben- oder Zwischenprodukte in einem oder mehreren separaten metabolischen Netze zusammenfassen. Dies ist vorteilhaft, wenn die erwarteten Ausscheide- oder Aufnahmeraten sich in unterschiedlichen Größenordnungen bewegen oder bestimmte Stoffwechselvorgänge in unterschiedlichen Detailgraden betrachtet werden sollen. Alternativ zur einem integrierten Modell kann mit den Schritten a) bis j) ein separates Modell zur Berechnung der Produktmerkmale erzeugt werden, dass den Prozessverlauf der externen Komponenten des separaten metabolischen Netzwerkes ebenfalls mit einem Satz von Makroreaktionen mit eigenen Kinetiken beschreibt. Neben- oder Zwischenprodukte, die nicht außerhalb des Organismus vorliegen, aber deren zellinterne Akkumulation sich auf ein- oder mehrere Produktmerkmale auswirkt können in Schritt a) und b) bei der Berechnung der EMs und der Formulierung der Makroreaktionen externalisiert, also als externe Komponenten klassifiziert, werden. Die Einbindung von Produktmerkmalen, die von zellinternen oder zellexternen Konzentrationen abhängig sind kann dann über die zusätzliche Integration von quantitativen oder qualitativen Zusammenhängen zwischen Konzentrationen und Produktmerkmalen erfolgen.

Weitere Gegenstände sind ein Computerprogramm oder eine Software zur Durchführung des erfindungsgemäßen Verfahrens.

Das mit dem erfindungsgemäßen Verfahren bereitgestellte Modell kann zur Prozessführung oder Planung der Prozessführung sowie Untersuchung des Verfahrens im Reaktor verwendet werden.

Eine besondere Ausführungsform des erfindungsgemäßen Verfahrens wird exemplarisch beschrieben, ohne sich darauf zu beschränken. Mit diesem Verfahren wurde ebenfalls exemplarisch ein Modell einer Fermentation mit Hybridoma-Zellen erstellt und dessen Validität wie beschrieben geprüft.

**Beisipiel: Modellierung einer Hybridoma-Zellkultur**

**1 Schritt a)**

Das Hintergrundwissen in Form eines metabolischen Netzwerkes wurde aus der Veröffentlichung von Niu et al. (Metabolic pathway analysis and reduction for mammalian cell cultures-Towards macroscopic modeling. Chemical Engineering Science (2013) 102, S. 461-473. DOI: 10.1016/j.ces.2013.07.034.) entnommen. Das hier beschriebene metabolische Netzwerk einer tierischen Zelle enthält 35 Reaktionen, die 37 interne und externe Metaboliten verknüpfen (siehe Fehler! **Verweisquelle konnte nicht gefunden werden.).** In der Veröffentlichung ist die Reversibilität der Reaktionen nicht explizit angegeben. Stattdessen wurden die Daten zur "Metabolischen Flussanalyse" aus derselben Veröffentlichung ausgewertet und zur Identifizierung der irreversiblen Reaktionen verwendet.

Mit der stöchiometrische Matrix N, die die Stöchiometrie, d. h. die stöchiometrischen Koeffizienten, der internen Metabolite enthält, und den Informationen über die Reversibilität der Reaktionen wurden mit Hilfe von METATOOL 5.1 (Pfeiffer et al. METATOOL: for studying metabolic networks, Bioinformatics 199915 (3), S. 251-257.) alle Elementary Modes (EMs) des Netzwerkes berechnet. Die Zahl der EMs liegt hier bei über 300.000.

**2 Schritt b)**

Die Matrix mit den berechneten EMs $E$ wurde in Schritt a) gewonnen. Analog zur Matrix $N$, enthält die Matrix $N_p$ die Stöchiometrie, d. h. die stöchiometrischen Koeffizienten, der externen Metabolite. Mögliche Makroreaktionen des stöchiometrischen Netzwerks wurden in der Matrix $K$ mit Formel 7 zusammengefasst:

$$K = N_p \cdot E \qquad \text{(Formel 7)}$$

**3 Schritt c)**

Die Messdaten des Prozesses wurden aus Baughman et al. (On the dynamic modeling of mammalian cell metabolism and mAb production. In: Computers & Chemical Engineering (2010) 34 (2), S. 210-222.) entnommen, der verschiedene Messgrößen einer Fermentation von Hybridoma-Zellen über den Verlauf eines Batch-Prozess (vgl. **Fehler! Verweisquelle konnte nicht gefunden werden.)** angibt.

**4 Schritt d)**

Mit Hilfe von spline-interpolierten Messwerten aus c) wurden die Wachstums- und Absterberaten sowie die spezifischen Aufnahme- bzw. Ausscheideraten berechnet (vgl. **Fehler! Verweisquelle konnte nicht gefunden werden.).** Die Lysis wurde mit einem vordefinierten und in das Verfahren eingegebenen, über den Prozesszeitraum konstanten Lysis-Faktor $K_l$ = 0.1 einbezogen. Ein Shiften der Messdaten war nicht notwendig, da es sich hier um Daten eines Batch-Prozesses ohne weitere Zugaben handelt. Die Daten zeigen dementsprechend einen stetigen Verlauf da alle Konzentrationsänderungen von den Zellen- und nicht von Zugaben verursacht sind.

Zur Berechnung der Raten $\tilde{q}$ werden zusätzliche Informationen herangezogen. So konnte mit Hilfe der ebenfalls im Datensatz angegebenen gesamten Biomasse (BM in $\left[\frac{C-mol}{l}\right]$) und der gesamten Zellzahl ein durchschnittlicher C-mol Gehalt von $f_{C-mol,X_v} = 18.41 \left[\frac{C-mol}{10^9 cells}\right]$ errechnet werden. Die C-mol bezogene Wachstumsrate konnte nun mit Formel 8:

$$\tilde{\mu}\left[\frac{C-mol}{h \cdot 10^9 cells}\right] = \mu\left[\frac{1}{h}\right] \cdot f_{C-mol,X_v}\left[\frac{C-mol}{10^9 cells}\right] \qquad \text{(Formel 8)}$$

berechnet werden. Analog kann die C-mol bezogene Bildungsrate des Antikörpers abgeschätzt werden. Hierzu wurde die molare Zusammensetzung des Antiköpers zu $CH_{1.58}O_{0.31}N_{0.27}S_{0.004}$ mit einer formellen molaren Masse von $M_{mAb,C-mol} = 22.45 \frac{g}{mol}$ geschätzt. Hier wird angenommen, dass die molare Zusammensetzung einer durchschnittlichen molaren Zusammensetzung von Proteinen wie von Villadsen et al. (Bioreaction engineering principles (2011), Kapitel 3, Elemental and Redox Balances, S. 73, Springer Verlag, ISBN: 978-1-4419-9687-9) angegeben entspricht. Die molare Masse des gesamten Antikörpers wurde zu $M_{mAb} = 150.000 \frac{g}{mol}$ geschätzt. Die Bildungsrate des Antikörpers ergab sich dann aus der Formel:

$$\tilde{q}_{mAb}\left[\frac{C-mol}{h \cdot 10^9 cells}\right] = q_{mAb}\left[\frac{10^{-4}mol}{h \cdot 10^9 cells}\right] \cdot \frac{M_{mAb,C-mol}}{M_{mAb}} \cdot 10^4 \qquad \text{(Formel 9)}$$

Der zeitliche Verlauf der Raten $\tilde{q}(t)$ konnte dann zur Auswahl der Makroreaktionen herangezogen werden.

**5 Schritt e)**

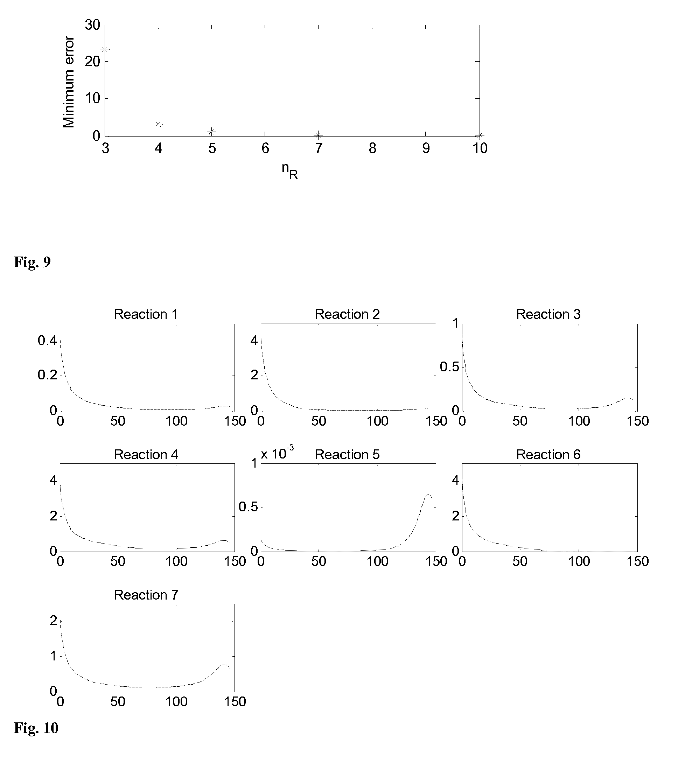

Im Schritt e) wurde ein kleines Set von Makroreaktionen erzeugt, mit dem der Datensatz bestmöglich wiedergegeben wurde. Hierzu wurde die Matrix *K* aus Schritt b) benötigt. Da die Anzahl von über 300.000 Makroreaktionen zu einer zu großen Anzahl von möglichen Kombinationen geführt hätte, wurde zuerst eine Vorreduktion durchgeführt.
Hierbei wurde ein Maximalwert für die Kosinus-Ähnlichkeit zweier Makroreaktionen von 0,995 definiert. Beginnend mit der ersten Reaktion wurden so alle Makroreaktionen aus der Matrix *K* entfernt, die diesen Wert überschritten. Es verblieben ca. 500 Makroreaktionen in der Matrix *K*, die weiterhin mehr als 95% des Originalvolumens des durch die EMs aufgespannten Lösungsraums abdecken.
Vor dem Auswahlprozess erfolgte zudem ein Abgleich der im metabolischen Netzwerk nach Niu et al. angegebenen Komponenten (die den externen Metaboliten des metabolischen Netzwerkes aus a) entsprechen) und den gemessenen Komponentenkonzentrationen aus c). Bis auf Prolin werden alle von Baughman et al. gemessenen Konzentrationen auch im metabolischen Netzwerk nach Niu et al. berücksichtigt. Um die Messung der Prolinkonzentration nutzen zu können wäre entweder ein anderes vereinfachtes Netzwerk, das Prolin als externen Metaboliten enthält, verwendbar oder eine Erweiterung des bestehenden metabolischen Netzwerks möglich.
Komponenten, die zwar in den berechneten Makroreaktionen vorkamen, aber von denen keine Daten verfügbar waren, wurden ebenfalls im Folgenden ignoriert. Die entsprechenden Zeilen der Matrix *K* wurden dementsprechend aus der Matrix gestrichen. Das Streichen der entsprechenden Zeilen bedeutet nicht, dass diese Ein- bzw. Ausgänge von der Zelle nicht genutzt werden. Sie bestehen weiterhin im metabolischen Netzwerk, nur fehlen hierzu Messungen, die diese bilanzierbar machen können. In diesem Beispiel wurden die Ein- bzw. Ausgänge von Arginine, Glutamate, Glycin, Histidin, Leucin, Lysine, Methionin, Ammonium, Sauerstoff, Phenylalanin, Serin, Threonin, Tryptophan, Tyrosin und Valin vernachlässigt.
In der folgenden Schritten des Verfahrens wird nun die reduzierte Matrix *K* - die die das Hintergrundwissen abbildet - und die Raten $\tilde{q}(t)$ aus d) sowie die Messdaten aus c) - die das Prozesswissen bilden - dazu verwendet, ein möglichst kleines Subset *L* der Makroreaktionen aus *K* zu gewinnen.
Bei einer Verwendung der Methode von Provost (Agnes Provost et al.) oder Soons (Zita Soons, Eugenio Ferreira, Isabel Rocha) werden die in d) berechneten Raten $\tilde{q}(t)$ benötigt. Erfindungsgemäß wurde hier hingegen die "lineare Schätzung von Reaktionsraten ausgewählter Makroreaktionen" zur Auswahl verwendet. Hierfür wurden lediglich die Messdaten verwendet. Ein Shiften ist wie oben erläutert nicht notwendig.
Analog zu den Raten $\tilde{q}(t)$ wurden die Messwerte der Zellzahl und des Antikörpers hier auf C-mol normiert. Dies ist erforderlich damit die Dimension der Makroreaktionen mit denen der Messwerte übereinstimmt.
Die Auswahl des Subsets erfolgte mit einem genetischen Algorithmus. In der Berechnung der Zielfunktion dieses genetischen Algorithmus wurde hierbei das bei der "lineare Schätzung von Reaktionsraten ausgewählter Makroreaktionen" angesprochene lineare Optimierungsproblem gelöst. Die hier errechnete finale Summe der kleinsten Fehlerquadrate des linearen Optimierungsproblems war zugleich der Wert der Zielfunktion für die jeweilige Auswahl der Makroreaktionen.
Zur Auswahl der Größe des Subsets *L* aus *K* wurde die Optimierung mit wiederholt mit einer unterschiedlichen Anzahl an Makroreaktionen in L durchgeführt. Die Anzahl stellt einen Kompromiss zwischen der Modellkomplexität und der Wiedergabegenauigkeit dar. Um zu ermitteln, wie viele Reaktionen ausreichend für die Wiedergabe sind, kann entweder die Auswahl des Subsets L für eine variierende Anzahl von Makroreaktionen wiederholt werden oder ein Strafterm für die Reaktionsanzahl wird direkt der Zielfunktion des genetischen Algorithmus hinzugefügt werden. In diesem Fall wurden mehrere Optimierungen mit einer vordefinierten Anzahl von Makroreaktionen (10, 7, 5, 4 und 3) durchgeführt. Die mit dem genetischen Algorithmus gefundene kleinste Summe der Fehlerquadrate ist in Figur 9 gegenüber der Zahl der Makroreaktionen aufgetragen. Es zeigte sich, dass in diesem Fall weniger als sieben Makroreaktionen zu wenig sind um den Prozessverlauf hinreichend gut darzustellen. Die gewählten Makroreaktionen sind in Tabelle 1 angegeben.

**Tabelle 1: Gewähltes Subset der Makroreaktionen (L). Nicht unterstrichene Komponenten werden in dem Modell nicht berücksichtigt, da hierzu keine Messungen vorliegen.**

0.474 *Alanine*+ 0.474 *Methionine*

$\rightarrow$ 0.158 *Asparagine* + 0.316 *Aspartate* + 0.632 *Glycine*

+ 0.158 *Tryptophan*

0.015 *A lanine*+ 0.00789 *Arginine* + 0.0304 *Asparagine* + 0.0161 *Glucose*

+ 0.0236 *Glutamine* + 0.00375 *Glutamate* + 0.00366 *Histidine*

+ 0.00953 *Isoleucine* + 0.015 *Leucine* + 0.112 *Methionine*

+ 0.00626 *Phenalanine* + 0.0154 *Serine* + 0.0109 *Valine*

$\rightarrow$ 0.963 *X* (*Biomass*) + 0.00276 *Aspartate* + 0.24 *Glycine*

+ 0.0208 *Tryptophan*

0.295 *Asparagine* + 0.147 *Glutamate*

$\rightarrow$ 0.295 *Aspartate* + 0.885 *Glycine* + 0.147 *Lactate*

0.00753 *Arginine* + 0.113 *Asparagine* + 0.0603 *Glucose* + 0.0225 *Glutamine*

+ 0.0824 *Histidine* + 0.00909 *Isoleucine* + 0.00597 *Phenalanine*

+ 0.0216 *Tryptophan* + 0.00431 *Tyrosine* + 0.0104 *Valine*

$\rightarrow$ 0.918 *X* (*Biomass*) + 0.061 *Alanine* + 0.0865 *Aspartate*

+ 0.343 *Glycine* + 0.0631 *Methionine*

0.0654 *Arginine* + 0.412 *Aspartate* + 0.00991 *Glucose* + 0.0145 *Glutamine*

+ 0.554 *Glycine* + 0.00226 *Histidine* + 0.00588 *Isoleucine*

+ 0.00926 *Leucine* + 0.00706 *Lysine* + 0.0649 *Phenalanine*

+ 0.0095 *Serine* + 0.00671 *Valine*

$\rightarrow$ 0.594 *X* (*Biomass*) + 0.049 *Alanine* + 0.395 *Asparagine*

+ 0.0503 *Threonine* + 0.0388 *Tryptophan*

0.0077 *Arginine* + 0.179 *Aspartate* + 0.0157 *Glucose* + 0.104 *Glutamine*

+ 0.216 *Glycine* + 0.00357 *Histidine* + 0.00929 *Isoleucine*

+ 0.0146 *Leucine* + 0.0112 *Lysine* + 0.038 *Tyrosine* + 0.0106 *Valine*

$\rightarrow$ 0.939 *X* (*Biomass*) + 0.0624 *Alanine* + 0.152 *Asparagine*

+ 0.0183 *Tryptophan*

0.0342 *Arginine* + 0.211 *Aspartate* + 0.00762 *Glucose* + 0.0195 *Glutamine*

+ 0.244 *Glycine* + 0.00452 *Histidine* + 0.0546 *Isoleucine*

+ *0.0185Leucine* + 0.0171 *Lysine* + 0.00406 *Methionine*

+ 0.0178 *Tyrosine* + 0.0203 *Valine*

$\rightarrow$ 0.457 *X* (*Biomass*) + 0.804 *IgG* (*Antibody*) + 0.185 *Asparagine*

+ 0.0153 *Tryptophan*

In den gezeigten Makroreaktionen sind alle externen Metaboliten des metabolischen Netzwerkes aus a) angegeben. Teil des Modells sind jedoch nur die unterstrichenen externen Metabolite, da nur für diese Messdaten aus c) vorliegen.

**6 Schritt f)**

Für das gewählte Set der Makroreaktionen wurden die Reaktionsraten über der Zeit ermittelt. Analog zu Schritt e) kann hier die Methode von Soons (Zita Soons, Eugenio Ferreira, Isabel Rocha) verwendet werden. In diesem Beispiel wurden mit der erfindungsgemäßen Methode "lineare Schätzung von Reaktionsraten ausgewählter Makroreaktionen" die in Figur 10 gezeigten Messwerte durch eine Schätzung der Reaktionsraten $r(t)$ approximiert. Das Ergebnis der Methode ist ein stückweise linearer Verlauf der einzelnen (volumetrischen) Reaktionsraten. Durch Division mit dem interpoliertem Verlauf der vitalen Zellzahl $X_v(t)$ wurden die zellspezifischen Reaktionsraten r(t) der in Tabelle 1 gezeigten Makroreaktionen gewonnen. Die so erhaltenen Reaktionsraten $\underline{r}(t)$ sind in Figur 10 abgebildet.

**7 Schritt g)**

Für alle in Tabelle 1 gezeigten Makroreaktionen wurden Reaktionskinetiken gemäß der Formel 10

$$\hat{r}_i(t) = r_{i,max} \cdot \prod_{k=1}^{K_i} \tilde{r}_k \left( \underline{c}(t), \underline{p}, \dots \right) \qquad \text{(Formel 10)}$$

angenommen, wobei $r_{i,max}$ die maximale Reaktionsgeschwindigkeit, $K_i$ die Anzahl der berücksichtigten Limitierungen und $\tilde{r}_k$ den Wert der einzelnen Limitierung k. Die Limitierung $\tilde{r}_k$ hängt der von einzelnen Konzentrationen $\underline{c}(t)$, dem Wert der Modellparameter $\underline{p}$ sowie weiteren Einflüssen ab. Durch die Reaktionsstöchiometrie vorgegeben sind z. B. Substratlimitierungen, die in diesem Fall mit einem Monod-Typ realisiert wurden. D. h. das bei jeder Reaktion i für jedes Substrat s dieser Reaktion, eine Limitierung gemäß Formel 11:

$$\tilde{r}_k = \left( \frac{[S]}{K_{m,i,s} + [S]} \right)^n \qquad \text{(Formel 11)}$$

eingeführt wurde. Hierbei ist [S] die Substratkonzentration, $K_{m,i,s}$ und $n$ sind Parameter, deren Werte in den Schritten h) und j) angepasst werden.

Weitere Terme ergeben sich aus der Analyse der Reaktionsraten r(t) aus f). In diesem Beispiel wurden neben Substratlimitierungen auch Inhibierungen gemäß Formel 12 berücksichtigt.

$$\tilde{r}_k = \left( \frac{K_{I,i,I}}{K_{I,i,I} + [I]} \right)^n \qquad \text{(Formel 12)}$$

Auch für diese Limitierung mussten die Werte der Parameter $K_{I,i,I}$ und $n$ angepasst werden. Die verwendeten kinetischen Terme der Reaktionen sind in Tabelle 2 angegeben.

**Tabelle 2: Kinetische Terme der gewählten Makroreaktionen aus L**

$$\hat{r}_1(t) = r_{1,max} \cdot \left( \frac{[Ala](t)}{K_{m,Ala,1} + [Ala](t)} \right) \cdot \left( \frac{[Glc](t)}{K_{m,Glc,1} + [Glc](t)} \right)^2 \cdot \left( \frac{K_{I,Asn,1}}{K_{I,Asn,1} + [Asn](t)} \right)^2$$

$$\hat{r}_2(t) = r_{2,max} \cdot \left( \frac{[Glc](t)}{K_{m,Glc,2} + [Glc](t)} \right) \cdot \left( \frac{[Gln](t)}{K_{m,Gln,2} + [Gln](t)} \right) \cdot \left( \frac{[Asn](t)}{K_{m,Asn,2} + [Asn](t)} \right)$$

$$\cdot \left( \frac{[Ala](t)}{K_{m,Ala,2} + [Ala](t)} \right)^2$$

$$\hat{r}_3(t) = r_{3,max} \cdot \left( \frac{[Glc](t)}{K_{m,Glc,3} + [Glc](t)} \right) \cdot \left( \frac{[Asn](t)}{K_{m,Gln,3} + [Asn](t)} \right) \cdot \left( \frac{K_{I,Lac,3}}{K_{I,Lac,3} + [Lac](t)} \right)^2$$

$$\hat{r}_4(t) = r_{4,max} \cdot \left( \frac{[Glc](t)}{K_{m,Glc,4} + [Glc](t)} \right) \cdot \left( \frac{[Gln](t)}{K_{m,Gln,4} + [Gln](t)} \right) \cdot \left( \frac{[Asn](t)}{K_{m,Asn,4} + [Asn](t)} \right)$$

$$\cdot \left( \frac{[X_t](t)}{K_{m,Xt,4} + [X_t](t)} \right)$$

(fortgesetzt)

$$\hat{r}_5(t) = r_{5,max} \cdot \left( \frac{[Glc](t)}{K_{m,Glc,5} + [Glc](t)} \right) \cdot \left( \frac{[Gln](t)}{K_{m,Gln,5} + [Gln](t)} \right) \cdot \left( \frac{[Asp](t)}{K_{m,Asp,5} + [Asp](t)} \right)$$

$$\cdot \left( \frac{K_{I,Asp,5}}{K_{I,Asp,5} + [Asp](t)} \right)^3 \cdot \left( \frac{K_{I,Asn,5}}{K_{I,Asn,5} + [Asn](t)} \right)$$

$$\hat{r}_6(t) = r_{6,max} \cdot \left( \frac{[Glc](t)}{K_{m,Glc,6} + [Glc](t)} \right) \cdot \left( \frac{[Gln](t)}{K_{m,Gln,6} + [Gln](t)} \right) \cdot \left( \frac{[Asp](t)}{K_{m,Asp,6} + [Asp](t)} \right)^2$$

$$\hat{r}_7(t) = r_{7,max} \cdot \left( \frac{[Glc](t)}{K_{m,Glc,7} + [Glc](t)} \right) \cdot \left( \frac{[Gln](t)}{K_{m,Gln,7} + [Gln](t)} \right) \cdot \left( \frac{[Asp](t)}{K_{m,Asp,7} + [Asp](t)} \right)$$

**8 Schritt h)**

Für jede Reaktionsrate konnte mit der in Tabelle 2 angegebenen Kinetik und den interpolierten Werten der in der Kinetik berücksichtigten Konzentrationen $\underline{C}^{int}(t)$ der Verlauf der Reaktionsrate $\hat{r}_i(\underline{p}, C^{int}(t))$ algebraisch berechnet werden.

Die Anpassung der Parameter dieser Kinetiken erfolgte separat für jede Reaktion i an die in Schritt f) ermittelte Reaktionsrate $r_i(t)$. Die Zielfunktion für eine Optimierung der in Reaktion *i* vorkommenden Parameter lautete in diesem Beispiel:

$$\min_{\underline{p_i}} \left( \sum_{k=0}^{T} \left( \hat{r}_i \left( \underline{p_i}, \underline{C}(t_k) \right) - r_i(t_k) \right)^2 \right) \qquad \text{(Formel 13)}$$

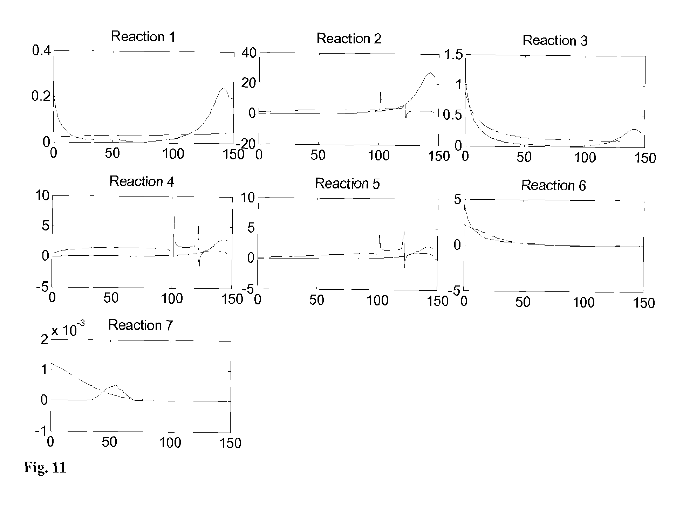

Die so angepassten Verläufe aller berechneten $\hat{r}_i(\underline{p}, C^{int}(t))$ sind zusammen mit den entsprechenden $r_i(t)$ in Figur 11 dargestellt. Die Verläufe der ersteren sind gestrichelt, die der letzteren durchgehend dargestellt. Es ist erkennbar, dass der qualitative Verlauf übereinstimmt. Dies bedeutet, dass mit den gewählten Kinetiken auch die Dynamik des Prozesses zufriedenstellend wiedergegeben werden kann. Diese Information ist in diesem Modellierungsschritt sehr hilfreich, da bei einer nicht zufriedenstellenden Wiedergabe die schnell durchzuführenden Schritte g) (Auswahl anderer Kinetiken) und h) (Parameterwertschätzung) wiederholt werden können, bis der gewünschte Anpassungsgrad erreicht ist. Es war hier keine Wiederholung (Schritt i)) erforderlich.

**9 Schritt j)**

Die weiterführende Anpassung der Modelparameterwerte erfolgte mit den Messdaten aus c). Hierfür wurden alle Parameter zugleich optimiert. Zudem wurden die bisher nicht betrachteten Vorgänge Apoptose und Lysis mit einbezogen. Diese werden in den Differentialgleichungen, die die Entwicklung der vitalen- und gesamten Zellzahl beschreiben, benötigt:

$$\frac{dX_v}{dt} = (\mu_x - \mu_d) \cdot X_v \qquad \text{(Formel 14)}$$

$$\frac{dX_t}{dt} = \mu_x \cdot X_v - K_l \cdot (X_t - X_v) \qquad \text{(Formel 15)}$$

Die gewählte Kinetik für die Beschreibung der Apoptose lautete:

$$\mu_d(t) = \mu_{d,max} \cdot \frac{\big([Lac](t) - C_{Lac,cr}\big)}{K_{d,Lac} + \big([Lac](t) - C_{Lac,cr}\big)} \qquad , [Lac] \geq C_{Lac,cr} \qquad \text{(Formel 16)}$$

$$\mu_d(t) = 0 \qquad\qquad\qquad\qquad , [Lac] < C_{Lac,cr} \qquad \text{(Formel 17)}$$

Der Lysis-Faktor $K_l$ wurde als konstant über dem Prozess angenommen. Neben den Parametern der Reaktionsraten wurden in diesem Schritt die durch die Apoptose und Lysis eingeführten Parameter bestimmt. In dem Beispiel wurde ausgehend von den Startwerten des Datensatzes durch numerisches Lösen des ODE-Systems der Verlauf der geschätzten Konzentrationen $\tilde{C}(t)$ ermittelt. Die Differenz zwischen den gemessenen Konzentrationen $\underline{C}^m(t)$ und den geschätzten Konzentrationen $\hat{\underline{C}}(t)$ wurde dabei mit üblichen Methoden mit folgender Zielfunktion minimiert:

$$\min_{\underline{p}} \left( \sum_{j=1}^{n_{comp}} \left( \sum_{k=0}^{T} \left( \hat{C}_j\big(\underline{p}, t_k\big) - C_j^m(t_k) \right)^2 \right) \right) \qquad \text{(Formel 18)}$$

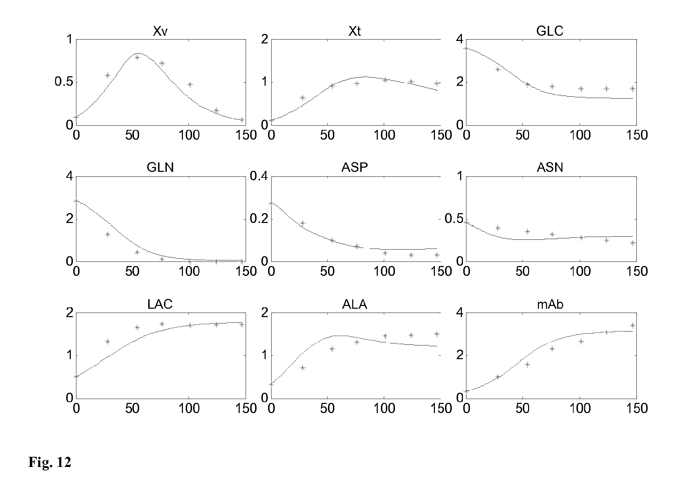

Mit insgesamt 33 Parametern $\underline{p}$ ist diese Optimierung i. d. R schwierig durchzuführen, da die Zielfunktion viele lokale Optima besitzt. Startet man einen deterministischen Optimierungsalgorithmus, wie z. B. den Levenberg-Marquardt Algorithmus an den aus Schritt h) bekannten Startwerten der Parameter ist die Erfolgsaussicht hingegen stark erhöht. Der angepasste Prozessverlauf ist in Figur 12 abgebildet. Die angepassten Parameter sind in Tabelle 3 abgebildet.

**Tabelle 3: Parameter der Kinetiken sowie der Apoptose und Lysis**

| | | | |
|---|---|---|---|
| $K_{m,Glc,1}$ | 14,6 | $K_{m,Gln,7}$ | 0,0187 |
| $K_{m,Ala,1}$ | 3,41 | $K_{m,Asp,7}$ | 0,872 |
| $K_{m,Glc,2}$ | 0,0508 | $r_{1,max}$ | 9,47 |
| $K_{m,Gln,2}$ | 0,00881 | $r_{2,max}$ | 9,91 |
| $K_{m,Asn,2}$ | 1,38 | $r_{3,max}$ | 57,6 |
| $K_{m,Ala,2}$ | 2,19 | $r_{4,max}$ | 21,7 |
| $K_{m,Glc,3}$ | 7,13 | $r_{5,max}$ | 0,345 |
| $K_{m,Asn,3}$ | 6,84 | $r_{6,max}$ | 49,4 |
| $K_{m,Xt,4}$ | 0,0315 | $r_{7,max}$ | 3,03 |
| $K_{m,Glc,4}$ | 1,29 | $K_{IAsn,1}$ | 16,1 |
| $K_{m,Gln,4}$ | 2,19 | $K_{I,Lac,3}$ | 0,681 |
| $K_{m,Asn,4}$ | 1,68 | $K_{I,Asp,5}$ | 9,74 |
| $K_{m,Glc,5}$ | 100 | $K_{I,Asn,5}$ | 1,10 |
| $K_{m,Gln,5}$ | 28,2 | $\mu_{d,max}$ | 0,125 |
| $K_{m,Asp,5}$ | 102 | $K_{d,Lac}$ | 1,01 |
| $K_{m,Glc,6}$ | 0,0451 | $C_{Lac,cr}$ | 1,22 |
| $K_{m,Gln,6}$ | 0,791 | $K_l$ | 0,00843 |
| $K_{m,Asp,6}$ | 1,06 | $K_{m,Glc,7}$ | 0,0145 |

**10 Schritt I)**

Das Modell, bestehend aus der Matrix $L$, den Kinetiken aus Tabelle 2 sowie den Kinetiken der Apoptose mit den mit den dazugehörigen Parameterwerten aus Tabelle 3 wurde ausgegeben.

**Symbolverzeichnis**

| _ (Unterstrich) | Bezeichnet einen Vektor |
|---|---|
| $_i$ (Index $i$) | Bezeichnet das i-te Element eines Vektors |
| $_k$ (Index k) | Bezeichnet das k-te Element eines Vektors |
| [ ] | Bezeichnet die Konzentration der in der Klammer stehenden Komponente |
| $C$ | Konzentration |
| $C^{Int}$ | Interpolierte Konzentration |
| $\hat{C}$ | Geschätzte Konzentration (z.B. durch das Lösen einer Differentialgleichung) |
| $C_s$ | Geshiftete Konzentration |
| $C_{cr}$ | Kritische Konzentration |
| $C^m$ | Gemessene Konzentration |
| $D$ | Verdünnungsrate |
| $q$ | Ermittelte zellspezifischen Ausscheide- und Aufnahmerate |
| $\tilde{q}$ | Ermittelte zellspezifischen Ausscheide- und Aufnahmerate, die von einer beliebigen Einheit auf $\left[\frac{Stoffmenge}{Zeit \cdot Zellzahl}\right]$ umgerechnet wurde $Zeit \cdot Zellzahl$ |
| r | Ermittelte Reaktionsrate |
| $\hat{r}$ | Geschätzte Reaktionsrate (z.B. durch das Berechnen einer Reaktionskinetik) |
| $\hat{r}$ | Limitierung einer Kinetik |
| $r_{max}$ | Parameter einer Reaktionskinetik |
| $N$ | Stöchiometrische Matrix |
| $N_p$ | Externe stöchiometrische Matrix |
| $K$ | Matrix, die Makroreaktionen enthält |
| $E$ | Matrix, die alle Elementary Modes enthält |
| $X_t$ | Gesamtzellzahl |
| $X_v$ | Vitale Zellzahl |
| $\mu$ | Wachstumsrate |
| $\mu_d$ | Absterberate |
| $\tilde{\mu}$ | Wachstumsrate, die von einer beliebigen Einheit auf $\left[\frac{Stoffmenge}{Zeit \cdot Zellzahl}\right]$ umgerechnet wurde |
| $K_d$ | Lysis-rate |
| $K_I$ | Parameter einer Inhibierungs-Limitierung |
| $K_M$ | Parameter einer Substratlimitierung |
| $n$ | Parameter einer Inhibierungs- oder Substratlimitierung |
| $L$ | Subset der Makroreaktionen, das für das Modell verwendet wird |
| $p$ | Modellparameter |
| $S$ | Substrat |
| $I$ | Inhibitor |

**Beschreibung der Figuren:**

Figur 1 zeigt das Shiften von Messdaten: Dargestellt ist der tatsächliche Verlauf einer gemessenen Größe ($c_i(t)$), der sich bei Änderungen der Verdünnungsrate ($D(t)$) sprunghaft ändert. Der geshiftete Verlauf ($c_{i,s}(t)$) kommt nur durch von der Zelle verursachte Änderungen zu Stande.

Figur 2 zeigt die Flux Map zweier spezifischer Raten $q_1$ und $q_2$. Die Höhenlinien geben die Häufigkeit an, mit der die jeweilige Kombination der Raten in den gemessenen Daten vorkommt.

Figur 3 zeigt eine dreidimensionale Darstellung des Lösungsraumes, der durch eine positive Linearkombination von EMs aufgespannt wird. In schwarz ist der Lösungsraum des gesamten Sets, in grau der eines Subsets dargestellt.

Figur 4 zeigt die Flux Map zweier spezifischer Raten $q_1$ und $q_2$. Als Vektoren sind die 2-dimensionalen Projektionen der Makroreaktionen eines Sets **L** dargestellt.

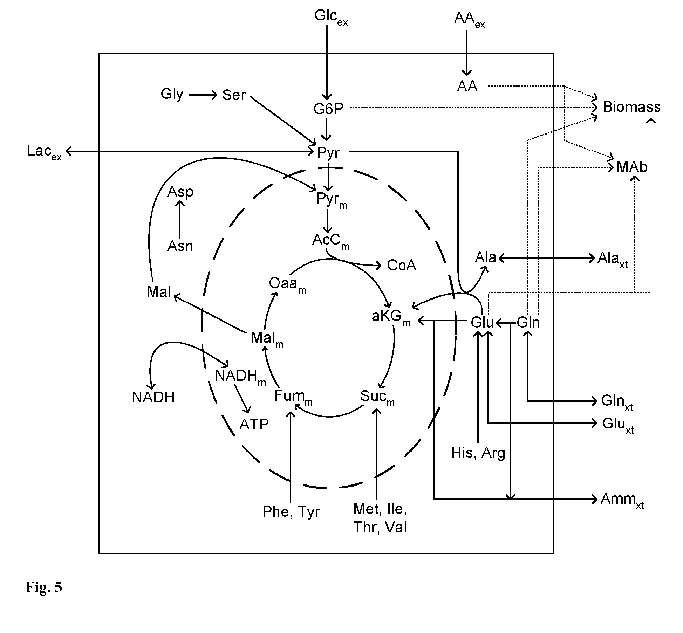

Figur 5 zeigt eine schematische Darstellung des Metabolischen Netzwerkes aus Niu et al. Hierbei ist die Begrenzung der Zelle als Kasten gezeigt. Die zellinterne Abgrenzung des Mitochondriums ist mit einer gestrichelten Linie gekennzeichnet. Externe Komponenten sind mit dem Index "xt" gekennzeichnet. Die Pfeile und gepunkteten Pfeile kennzeichnen Reaktionen.

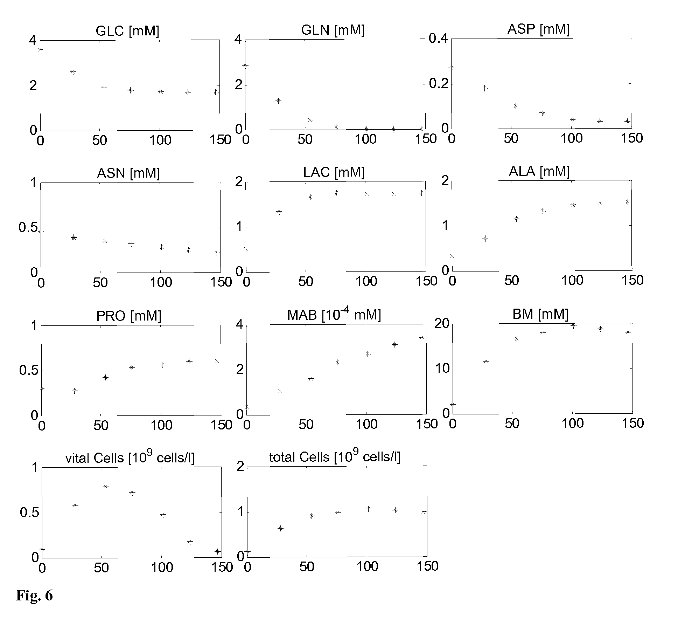

Figur 6 zeigt die Messdaten einer Fermentation mit Hybridoma-Zellen aus Baughman et al. Die Gesamtzellzahl (total Cells) ist hierbei aus der Summe der lebenden Zellen (vital Cells) und toten Zellen (dead Cells) berechnet. Die Abkürzungen GLC, GLN, ASP, ASN, LAC, ALA und PRO bezeichnen das Substrat Glucose und die Aminosäuren Glutamin, Asparaginsäure, Asparagin, Alanin und Prolin sowie das Stoffwechselprodukt Lactat. Die Abkürzung MAB bezeichnet das Produkt monoklonaler Antikörper und BM die Biomasse.

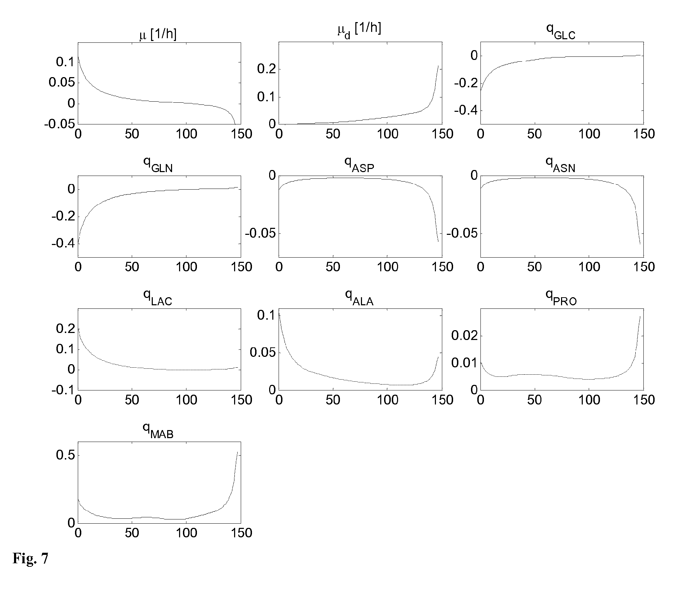

Figur 7 zeigt die Wachstums- und Absterberaten sowie die zellspezifischen Aufnahme- und Ausscheideraten. Alle zellspezifischen Raten bis auf $q_{MAB}$ sind in $\left[\frac{mM}{h \cdot 10^9 Cells}\right]$ angegeben. Die Rate $q_{MAB}$ ist in $\left[\frac{10^{-4}mM}{h \cdot 10^9 Cells}\right]$ angegeben.

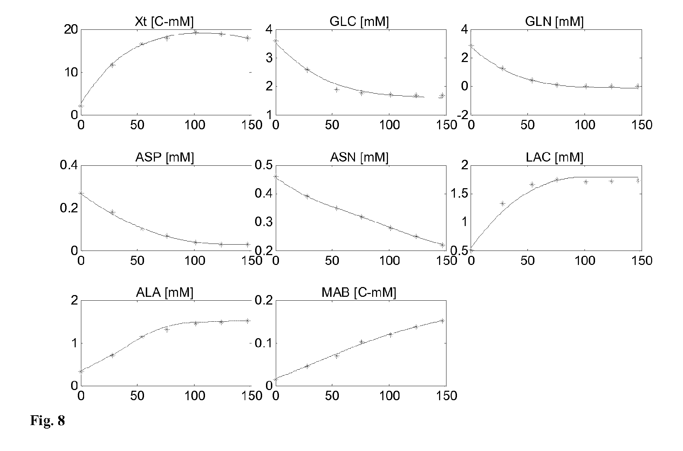

Figur 8 zeigt die mit der "linearen Schätzung von Reaktionsraten ausgewählter Makroreaktionen" mit dem gewählten Reaktionsset approximierten Konzentrationen. Die Gesamtzellzahl ($X_t$) sowie die Antikörperkonzentration (**MAB**) wurden dafür auf C-mol umgerechnet.

Figur 9 zeigt die kleinste ermittelte Summe der Fehlerquadrate ("Minimum error") aufgetragen über der Zahl der Makroreaktionen im Subset ($n_R$).

Figur 10 zeigt die mit der erfindungsgemäßen Methode "lineare Schätzung von Reaktionsraten ausgewählter Makroreaktionen" ermittelten Reaktionsraten der Makroreaktionen $\underline{r}(t)$.

Figur 11 zeigt die mit der erfindungsgemäßen Methode "lineare Schätzung von Reaktionsraten ausgewählter Makroreaktionen" ermittelten Reaktionsraten der Makroreaktionen $\tilde{r}(t)$ (durchgehende Linie) zusammen mit den algebraisch berechneten Reaktionsraten $\hat{r}(\underline{p}, C^{int}(t))$ (gestrichelte Linie)

Figur 12 zeigt einen Vergleich der gemessenen Konzentrationen $\underline{C}^m(t)$ (Punkte) und dem simuliertem Prozessverlauf C(t) (durchgehende Linie). Die Konzentrationen sind in **[mM]** angegeben. Ausnahmen sind die vitale und gesamte Zellzahl ($X_v/X_t$ in **[$10^9$ cells /l]**) und die Konzentration des Antikörpers (**mAb** in [$10^{-4}$ **mM**])*.

**Patentansprüche**

1. Computerimplementiertes Verfahren zur Erstellung eines Models einer Bioreaktion mit einem Organismus, das folgende Schritte umfasst:

   a. Ausgewählte Stoffwechselwege des Organismus, deren Stöchiometrie- sowie Reversibilitätseigenschaften werden als Hintergrundwissen in das Verfahren eingegeben und Elementary Modes werden aus dieser Eingabe berechnet.
   b. Die Elementary Modes aus a) werden in einer Matrix $K$ zusammengefasst, wobei die Elementary Modes die Stoffwechselwege aus a) in Makroreaktionen zusammenfassen und die Matrix $K$ die Stöchiometrie und die Reversibilitätseigenschaften aller Makroreaktionen enthält.

c. Die Messdaten zur Bioreaktion mit dem Organismus werden eingegeben.

d. Mit Hilfe einer Interpolationsmethode werden auf Basis der eingegebenen Messdaten aus c) die für den Organismus spezifischen Raten - Ausscheide- und Aufnahmeraten von einer oder mehreren Eingangsgrößen und Ausgangsgrößen - der eingegebenen Stoffwechselwege berechnet.

e. Relevante Makroreaktionen werden in Form eines Subsets der Elementary Modes aus a) ausgewählt durch

    i. datenunabhängige Vorreduktion der Anzahl der Elementary Modes aus a),

    ii. Auswahl des Subsets basiert auf den Messdaten aus c) und / oder einer oder mehrerer Raten aus d), bevorzugt basierend auf einer oder mehreren der Raten aus d), aus der vorreduzierten Anzahl der EMs aus i.,

    iii. Das Subset wird in einer Matrix L zusammengefasst, die die Stöchiometrie und Reversibilität der ausgewählten Makroreaktionen des Subsets aus ii. beschreibt.

f. Mit Hilfe einer Interpolationsmethode werden auf Basis der eigegebenen Messdaten aus c) und / oder der Raten aus d), die Reaktionsraten der Makroreaktionen des Subsets berechnet.

g. Die Kinetiken der Makroreaktionen des Subsets werden auf Basis der Stöchiometrie der Makroreaktionen des Subsets aus e) iii. und einer individuellen Anpassung basierend auf einer Analyse der Reaktionsraten aus f) entworfen; dadurch werden die Modellparameter definiert.

h. Optional erfolgt aus den Kinetiken aus g) eine erste Anpassung der Modellparameterwerte an die berechneten Reaktionsraten aus f) für jede Makroreaktion separat.

i. Optional werden die Schritte g) und h) wiederholt bis eine vordefinierte Anpassungsgüte erreicht wird.

j. Die Modellparameterwerte werden an die Messdaten aus c) angepasst.

k. Die Matrix L, die Kinetiken aus g) und die Modellparameterwerte aus j) bilden das Modell und werden ausgegeben.

**2.** Computer-implementiertes Verfahren nach Anspruch 1, wobei im Schritt d) auch Wachstumsraten, besonders bevorzugt auch Absterberaten des Organismus berechnet werden.

**3.** Computer-implementiertes Verfahren nach einem der Ansprüche 1 oder 2, wobei im Schritt g) eine individuelle Anpassung der Kinetiken basierend auf einer Analyse der Reaktionsraten aus f) erfolgt.

**4.** Computer-implementiertes Verfahren nach einem der Ansprüche 1 bis 3, wobei im Schritt h) die Anpassung der Parameterwerte der Kinetiken aus g) für jede Makroreaktion separat durch Kombination von mehreren Anpassungsmethoden erfolgt.

**5.** Computer-implementiertes Verfahren nach einem der Ansprüche 1 bis 4, wobei im Schritt e) zur Auswahl des Subsets von Makroreaktionen nach Schritt ii. eine lineare Schätzung von Reaktionsraten ausgewählter Makroreaktionen durchgeführt wird.

**6.** Computer-implementiertes Verfahren nach einem der Ansprüche 1 bis 5, wobei im Schritt e) zur Auswahl des Subsets von Makroreaktionen nach Schritt ii. eine lineare Schätzung von Reaktionsraten ausgewählter Makroreaktionen in Kombination mit einem evolutionären Algorithmus durchgeführt wird.

**7.** Computer-implementiertes Verfahren nach einem der Ansprüche 1 bis 6, wobei die Messdaten vor Anwendung der Interpolationsmethode im Schritt d) geshiftet werden, um die Beschreibung eines konstanten Verbrauchs ohne Feedpeaks zu erreichen.

**8.** Computer-implementiertes Verfahren nach einem der Ansprüche 1 bis 7, wobei im Schritt f) eine lineare Schätzung von Reaktionsraten ausgewählter Makroreaktionen durchgeführt wird.

**9.** Computer-implementiertes Verfahren nach einem der Ansprüche 1 bis 8, wobei im Schritt e) die Validität der Auswahl des Subsets von Makroreaktionen nach Schritt ii. mit Hilfe einer Flux-Map geprüft wird.

**10.** Computerprogramm zur Durchführung der Verfahrensschritte nach einem der Ansprüche 1 bis 9.

**11.** Software zur Durchführung der Verfahrensschritte nach einem der Ansprüche 1 bis 10.

**Fig. 1**

Fig. 2

Fig. 3

Fig. 4

Fig. 5

Fig. 6

Fig. 7

Fig. 8

Fig. 9

Fig. 10

Fig. 11

Fig. 12

Europäisches Patentamt
European Patent Office
Office européen des brevets

**EUROPÄISCHER RECHERCHENBERICHT**

Nummer der Anmeldung

EP 15 15 3052

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | J. GAO ET AL: "Dynamic Metabolic Modeling for a MAB Bioprocess", BIOTECHNOLOGY PROGRESS, Bd. 23, Nr. 1, 2. Februar 2007 (2007-02-02), Seiten 168-181, XP055199374, ISSN: 8756-7938, DOI: 10.1021/bp060089y * Zusammenfassung * * Seite 169, Spalte 1, Absatz 3 - Seite 170, Spalte 1, Absatz 1 * * Seite 175, Spalte 1, Absatz 1 - Absatz 3 * | 1-11 | INV. G06F19/12 ADD. G06F19/26 |
| X | PROVOST A ET AL: "Metabolic design of macroscopic bioreaction models: application to Chinese hamster ovary cells", BIOPROCESS AND BIOSYSTEMS ENGINEERING, SPRINGER, BERLIN, DE, Bd. 29, Nr. 5-6, 30. September 2006 (2006-09-30), Seiten 349-366, XP019461015, ISSN: 1615-7605, DOI: 10.1007/S00449-006-0083-Y * Zusammenfassung * * Seite 350, Spalte 2, Absatz 1 - Seite 352, Spalte 2, Absatz 5 * * Abbildungen 1,6 * * Seite 171, Spalte 2, Absatz 3 - Seite 173, Spalte 1, Absatz 1 * | 1-11 | |

RECHERCHIERTE SACHGEBIETE (IPC)

G06F

Der vorliegende Recherchenbericht wurde für alle Patentansprüche erstellt

| Recherchenort | Abschlußdatum der Recherche | Prüfer |
|---|---|---|
| München | 3. Juli 2015 | Schmitt, Constanze |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **FRAHM B ; LANE P ; ATZERT H ; MUNACK A ; HOFFMANN M ; HASS VC ; PORTNER R.** Adaptive, Model-Based Control by the Open-Loop-Feedback-Optimal (OLFO) Controller for the Effective Fed-Batch Cultivation of Hybridoma Cells. *Biotechnol. Prog,* 2002, vol. 18 (5), 1095-1103
- **KONTORAVDI C ; ASPREY SP ; PISTIKOPOULOS EN ; MANTALARIS A.** Development of a dynamic model of monoclonal antibody production and glycosylation for product quality monitoring. *Computers & Chemical Engineering,* 2007, vol. 31 (5-6), 392-400
- **JEDRZEJEWSKI PM ; DEL VAL, IOSCANI JIMENEZ ; CONSTANTINOU A ; DELL A ; HASLAM SM ; POLIZZI KM ; KONTORAVDI C.** Towards Controlling the Glycoform: A Model Framework Linking Extracellular Metabolites to Antibody Glycosylation. *International journal of molecular sciences,* 2014, vol. 15 (3), 4492-4522
- **JIMENEZ DEL VAL ; IOSCANI, NAGY JM ; KONTORAVDI C.** A dynamic mathematical model for monoclonal antibody N-linked glycosylation and nucleotide sugar donor transport within a maturing Golgi apparatus. *Biotechnology progress,* 2011, vol. 27 (6), 1730-1743
- **LEIFHEIT J ; HEINE T ; KAWOHL M ; KING R.** Rechnergestützte halbautomatische Modellierung biotechnologischer Prozesse (Semiautomatic Modeling of Biotechnical Processes). *Automatisierungstechnik,* 2007, vol. 55 (5
- **PROVOST A.** Metabolic design of dynamic bioreaction models. Faculté des Sciences Appliquees, Universite catholique de Louvain, Louvain-la-Neuve. *Louvain-la-Neuve,* 2006, S. 81 ff, , S.107 ff. S. 118 ff
- **PFEIFFER T ; MONTERO F ; SCHUSTER S.** METATOOL: for studying metabolic networks. *Bioinformatics,* 1999, vol. 15 (3), 251-257
- **PROVOST A.** Metabolic design of dynamic bioreaction models. *Faculté des Sciences Appliquees,* 2006, 81
- **PROVOST A.** Metabolic design of dynamic bioreaction models. *Faculté des Sciences Appliquees,* 2006, S. 87, , S. 118 ff

- **GAO, J. et al.** Dynamic metabolic modeling for a MAB bioprocess. *Biotechnology progress,* 2007, vol. 23 (1), 168-181
- **LEIFHEIT, J. ; HEINE, T. ; KAWOHL, M. ; KING, R.** Rechnergestützte halbautomatische Modellierung biotechnologischer Prozesse (Semiautomatic Modeling of Biotechnical Processes). *Automatisierungstechnik,* 2007, vol. 55 (5), 211-218
- **PROVOST A.** Metabolic design of dynamic bioreaction models. *Faculté des Sciences Appliquees, Universite catholique de Louvain, Louvain-la-Neuve, Louvain-la-Neuve,* 2006
- **SOONS, Z. I. T. A. ; FERREIRA, E. C. ; ROCHA, I.** *Selection of elementary modes for bioprocess control,* 2010
- **LEIGHTY, R. W. ; ANTONIEWICZ, M. R.** Dynamic metabolic flux analysis (DMFA): a framework for determining fluxes at metabolic non-steady state. *Metabolic engineering,* 2011, vol. 13 (6), 745-755
- **SYED MURTUZA BAKER ; KAI SCHALLAU ; BJÖRN H. JUNKER.** Comparison of different algorithms for simultaneous estimation of multiple parameters in kinetic metabolic models. *J. Integrative Bioinformatics,* 2010, 1-1
- **PROVOST A.** Metabolic design of dynamic bioreaction models. *Faculté des Sciences Appliquees,* 2006, 126
- **GAO, J. ; GORENFLO, V. M. ; SCHARER, J. M. ; BUDMAN, H. M.** Dynamic metabolic modeling for a MAB bioprocess. *Biotechnology progress,* 2007, vol. 23 (1), 168-181
- **NIU et al.** Metabolic pathway analysis and reduction for mammalian cell cultures-Towards macroscopic modeling. *Chemical Engineering Science,* 2013, vol. 102, 461-473
- **PFEIFFER et al.** METATOOL: for studying metabolic networks. *Bioinformatics,* 1999, vol. 15 (3), 251-257
- **BAUGHMAN et al.** On the dynamic modeling of mammalian cell metabolism and mAb production. *Computers & Chemical Engineering,* 2010, vol. 34 (2), 210-222
- Bioreaction engineering principles. **VILLADSEN et al.** Elemental and Redox Balances. Springer Verlag, 2011, vol. 73